# EUROPEAN PATENT SPECIFICATION

(11) **EP 4 382 079 B1**
(45) Date of publication and mention of the grant of the patent: **05.11.2025**
(21) Application number: 23194331.7
(22) Date of filing: 30.08.2023
(51) Int. Cl.: A61F 2/16

(54) **ADAPTIVE INTRAOCULAR LENS HOLDER**
ADAPTIVER HALTER FÜR INTRAOKULARLINSEN
SUPPORT ADAPTATIF DE LENTILLE INTRAOCULAIRE

(30) Priority: 31.08.2022 US 202263374235 P
(43) Date of publication of application: 12.06.2024
(73) Proprietor: Johnson & Johnson Surgical Vision, Inc., Irvine, CA 92618 (US)
(72) Inventor: PEREZ, Yasemar, Irvine, 92618 (US); ANSELL, Scott, Irvine, 92618 (US)
(74) Representative: Carpmaels & Ransford LLP

(56) References cited:
- WO-A1-2020/121272
- WO-A1-95/24863
- WO-A2-2018/006889
- US-A- 6 129 733
- US-A- 6 142 999

## Description

### CROSS REFERENCE TO RELATED APPLICATIONS

This application claims priority to U.S. Provisional Application No. 63/374235, filed on August 31, 2022.

### FIELD OF TECHNOLOGY

The field of the disclosure relates generally to an intraocular lens (IOL) holder. More particularly, the disclosure relates to a holder, case, cassette, and/or package for holding, transporting, shipping, carrying, washing, sterilizing, aerating, degassing, and/or storing IOLs of various types in a variety of models, shapes, and sizes.

### BACKGROUND

Artificial IOLs can be used to replace the natural lens of the eye, e.g., for the treatment of cataracts, or can be used to correct vision while leaving the natural lens of the eye in place. There are several types of artificial IOLs that are commonly used in eye surgery that are available in a variety of models, shapes, and sizes, and that are manufactured from various materials.

Intraocular lenses are made from various biocompatible materials including, but not limited to, polymethylmethacrylate (PMMA), silicone polymers, acrylic polymers, hydrogel polymers, and the like. Different types of IOLs include, but are not limited to, monofocal, multifocal, accommodating, presbyopic-correcting, and toric lens implants. In addition to the lens body, commonly referred to as the optic, most IOLs also include haptic features that are primarily responsible for positioning and maintaining mechanical and rotational stability of the IOL implant in the eye. Intraocular lenses can be manufactured as single piece lenses, which are manufactured with the optic and the haptics together from the same material, or as multiple piece lenses, wherein the optic and the haptics may be made of different materials. The size, shape, and thickness of the optic as well as the size, shape and number of lens haptics varies among different types and models of intraocular lens.

Along with the development of many different types, models, shapes, and sizes of intraocular lenses being manufactured from various materials, various shapes, sizes, and designs of IOL holders have been developed. IOL holders carry out a variety of functions including, but not limited to, providing a means of protection and support for holding, transporting, shipping, carrying, washing, sterilizing, aerating, degassing, and storing IOLs.

Currently, most IOL holders are composed of several different materials; require multiple pieces and/or different inserts or parts to accommodate different types, models, shapes, and sizes of IOLs; and/or require specialized assembly, often required to be completed at manufacturing sites, which adds complexity to the components supply chain and manufacturing operations. A more universal IOL holder is needed that is adaptive to accommodate different types, models, shapes, and sizes of IOLs without requiring multiple components or specialized assembly.

US6142999A describes a folding device for holding and folding an IOL and adapted to allow a surgeon to select between loading the IOL into a removably attached IOL injector and between holding the IOL in a folded state for removal with a separate instrument. WO2018006889A2 describes a cartridge for an IOL injector system comprising a folder element. WO2020121272A1 describes a haptic optic management system which comprises a first cam assembly comprising a first cam body portion, an opening in the first cam body portion, and haptic folder arms disposed in the opening.

### BRIEF SUMMARY

The invention is defined by the appended independent claims. Certain embodiments are defined by the appended dependent claims. In one aspect, this disclosure relates to an IOL holder comprising a one-piece device comprising a base, a lid, and at least one integral hinge that connects the base to the lid, wherein the base comprises an integral annular support that accommodates an intraocular lens, wherein the lid comprises an integral adaptive feature that exerts a force on a lens body of an intraocular lens when an intraocular lens is positioned on the integral annular support of the base and the lid is in a closed position.

In another aspect, this disclosure relates to a method of manufacture of an IOL holder comprising forming an IOL holder as a one-piece device from at least one polymer, by molding or printing the IOL holder from the polymer, wherein the IOL holder comprises a one-piece device comprising a base, a lid, and at least one integral hinge that connects the base to the lid, wherein the base of the IOL holder comprises an integral annular support that is designed to accommodate an intraocular lens, and wherein the lid of the IOL holder comprises an integral adaptive feature that exerts a force on a lens body of an intraocular lens when an intraocular lens is positioned on the integral annular support of the base and the lid is in a closed position.

Also disclosed (but not recited in the claims) is a method of use of an IOL holder comprising holding, transporting, shipping, carrying, washing, sterilizing, aerating, degassing, and/or storing an intraocular lens in an IOL holder, wherein the IOL holder comprises a one-piece device comprising a base, a lid, and at least one integral hinge that connects the base to the lid, wherein the base of the IOL holder comprises an integral annular support that is designed to accommodate an intraocular lens, and wherein the lid of the IOL holder comprises an integral adaptive feature that exerts a force on a lens body of an intraocular lens when an intraocular lens is positioned on the integral annular support of the base and the lid is in a closed position.

### BRIEF DESCRIPTION OF THE DRAWINGS

The Figures described herein depict primarily generalized embodiments or data in support of generalized embodiments, which embodiments will be described with additional specificity and detail in connection with the drawings in which:
FIG. 1 is an illustration of an embodiment of the IOL holder disclosed herein displayed with a lid in an open position with a single piece intraocular lens in place on the integral annular support with the intraocular lens haptics in contact with the recess features of the integral posts.
FIG. 2 is an illustration of an embodiment of the IOL holder disclosed herein displayed with a lid in an open position with a multiple piece (3-piece) intraocular lens in place on the integral annular support with the intraocular lens haptics partially wrapped around the integral posts.
FIG. 3 is an illustration of an embodiment of the IOL holder disclosed herein displayed with a lid in a closed position.
FIG. 4 is an illustration of an embodiment of the IOL holder disclosed herein displayed with a lid in an open position with a single piece intraocular lens in place on the integral annular support with the intraocular lens haptics in contact with the recess features of the integral posts.
FIG. 5 is an illustration of an embodiment of the IOL holder disclosed herein displayed with a lid in a closed position.

### DETAILED DESCRIPTION

In the following specification and the claims, reference will be made to a number of terms, which shall be defined to have the following meanings. The singular forms "a," "an," and "the" include plural references unless the context clearly dictates otherwise. The terms "comprising," "including," and "having" are intended to be inclusive and mean that there may be additional elements other than the listed elements. "Optional" or "optionally" means that the subsequently described event or a circumstance may or may not occur, and that the description includes instances where the event occurs and instances where it does not.

Approximating language, as used herein throughout the specification and claims, may be applied to modify any quantitative representation that could permissibly vary without resulting in a change in the basic function to which it is related. Accordingly, a value modified by a term or terms, such as "about," "approximately," and "substantially," are not to be limited to the precise value specified unless the context clearly dictates otherwise. In at least some instances, the approximating language may correspond to the precision of an instrument for measuring the value. Here and throughout the specification and claims, range limitations may be combined and/or interchanged; such ranges are identified and include all the sub-ranges contained therein unless context or language indicates otherwise.

As used herein, the term "IOL holder" refers to any device that is designed to be used for one or more function including, but not limited to, holding, transporting, shipping, carrying, washing, sterilizing, aerating, degassing, and storing IOLs of various types, models, shapes, and sizes. The term "holder" may be used interchangeably with case, cassette, package, and related terms that refer to similar devices that perform similar functions.

As used herein, the terms "intraocular lens" and "IOL" refer to an artificial lens that is designed to be placed within the eye of a subject. The IOL includes both the lens body, i.e., the optic, and may also include haptic features. Intraocular lenses are made from various materials including, but not limited to, thermal plastic, polymethylmethacrylate (PMMA), silicone, and hydrophobic acrylic. Different types of IOLs include, but are not limited to, monofocal, multifocal, accommodating, presbyopic-correcting, and toric lens implants. Intraocular lenses can be manufactured as single piece lenses, which are manufactured with the optic and the haptics together from the same material, or as multiple piece lenses, wherein the optic and the haptics may be made of different materials. The size, shape, and thickness of the optic as well as the size, shape and number of lens haptics varies among different types and models of intraocular lens.

As used herein, the terms "lens body," and "optic" specifically refer to the portion of an IOL that is designed to refract light.

As used herein, the terms "haptic features" and "haptics" refer to the portion of an IOL that is specifically designed for positioning and maintaining mechanical and rotational stability of the IOL implant in the eye. Intraocular lenses may have one or more haptics, and the haptics may be manufactured from the same material as the optic, e.g., in single piece lenses, or the haptics may be made from a material that is different from the optic, e.g., for most multiple piece lenses. Each haptic may be joined with the lens at one connection, or at two with so-called loop-type haptics.

As used herein, the terms "using" refers to such methods of use comprising holding, transporting, shipping, carrying, washing, sterilizing, aerating, degassing, and/or storing, and the like.

As used herein, the term "subject" refers to a warm-blooded animal such as a mammal, which is to benefit from the replacement or insertion of at least one lens in the eye with an intraocular lens. It is understood that at least humans, dogs, cats, and horses are within the scope of the meaning of the term.

The present application discloses a one-piece IOL holder that is surprisingly adaptive to accommodate different types, models, shapes, and sizes of IOLs without requiring multiple parts or complex assembly. Further disclosed is a method of manufacture of an IOL holder that is surprisingly adaptive to accommodate different types, models, shapes, and sizes of IOLs. Further disclosed is a method of use of an IOL holder that is surprisingly adaptive to accommodate different types, models, shapes, and sizes of IOLs. The IOL holder, method of manufacture of the IOL holder, and method of use of the IOL holder disclosed herein provide benefits over the state of the art by reducing manufacturing complexities and costs, while increasing flexibility, efficiency, and effectiveness.

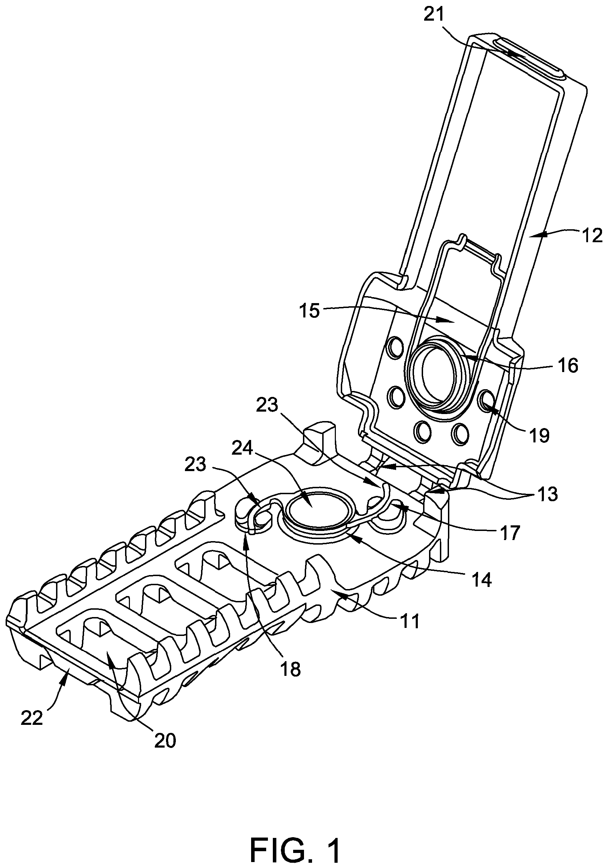

In various embodiments, the IOL holder comprises a one-piece device comprising a base, a lid, and at least one integral hinge that connects the base to the lid, wherein the base of the IOL holder comprises an integral annular support that is designed to accommodate an intraocular lens, and wherein the lid of the IOL holder comprises an integral adaptive feature that exerts a force on a lens body of an intraocular lens when an intraocular lens is positioned on the integral annular support of the base and the lid is in a closed position. Preferentially, as depicted in Figure 1, the base **11** and the lid **12** of the IOL holder are integrally connected by two integral hinges **13.**

In various embodiments, the IOL holder is manufactured from a polymer. Suitable polymers include those that are compatible with the material of construction of an intraocular lens and the methods of use of the IOL holder including, but not limited to, thermal plastics, biopolymers, and copolymers. More specifically, the polymers include, but are not limited to, polyolefins (e.g., polypropylene and polyethylene), polycarbonate, acrylic polymers, acrylic copolymers, nylon, polyester, cellulose acetate, acetate/butyrate, and the like. In one embodiment, a glassomer insert may be used. Preferably, the IOL holder comprises polypropylene or a thermal plastic.

In various embodiments, the IOL holder can accommodate various types, models, shapes, and sizes of a single piece or a multiple piece intraocular lens. In various embodiments, the IOL holder can accommodate various types, models, shapes, and sizes of a single piece intraocular lens. In various embodiments, the IOL holder can accommodate various types, models, shapes, and sizes of a multiple piece intraocular lens.

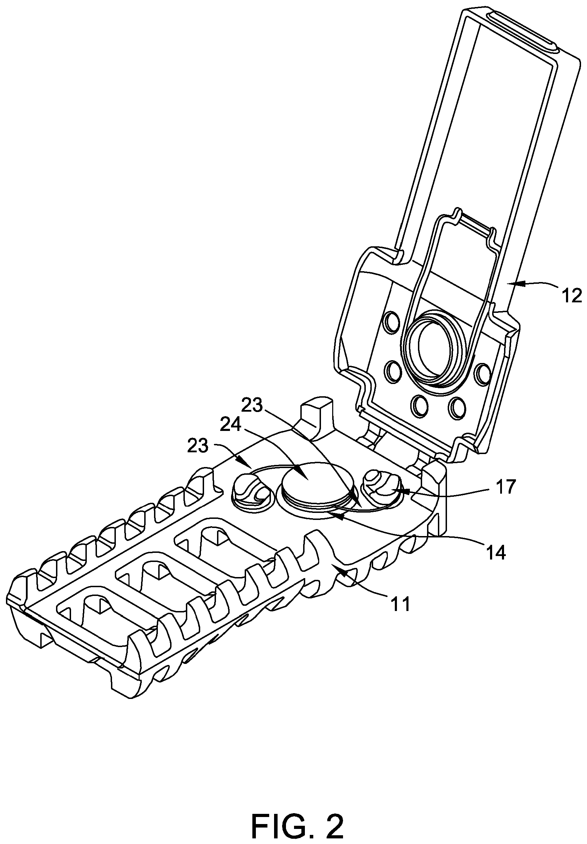

In various embodiments, the integral annular support is designed to protrude from the base of the IOL holder as a column comprising a concave profile at the distal end of the integral annular support with respect to the base, such that an intraocular lens may be positioned on the concave profile. In various embodiments, the integral annular support is designed to be level with the base of the IOL holder and comprise a concave profile in which an intraocular lens may be positioned. In various embodiments, the integral annular support is designed to recede within the base of the IOL holder and comprise a concave profile in which an intraocular lens may be positioned. In the device depicted in Figures 1 and 2, the integral annular support **14** protrudes from the base **11** in a columnar shape and comprises a concave profile at the distal end of the integral annular support **14** with respect to the base **11.**

**It** will be appreciated that the integral annular support **14** does not have to have a columnar shape base portion. The base of the support may be of any shape. By way of example, and not of limitation, the base of the integral annular support **14** may be oval, square, rectangular, pentagonal, hexagonal, etc. However, in one aspect of the invention the portion which contacts the optic portion of the intraocular lens will be annular in shape.

In various embodiments, the integral annular support 14 is designed to avoid significant contact with the optical surface of the lens. In further embodiments, the integral annular support is designed to avoid contact with the optical surface of the lens. In still further embodiments, the integral annular support is designed to avoid contact with the central portion of the optical surface of the lens within a 5 mm diameter. In still further embodiments, the integral annular support is designed to avoid contact with the central portion of the optical surface of the lens within a 3 mm diameter.

In various embodiments, the integral annular support is designed to avoid significant contact with the optical surface of the lens. In further embodiments, the integral annular support is designed to avoid contact with the optical surface of the lens. In still further embodiments, the integral annular support is designed to avoid contact with the central portion of the optical surface of the lens within a 5 mm diameter. In still further embodiments, the integral annular support is designed to avoid contact with the central portion of the optical surface of the lens within a 3 mm diameter.

In various embodiments, the IOL holder includes an integral adaptive feature that comprises a cantilever design, wherein only one end of the integral adaptive feature is integrally connected to the lid. In various embodiments, the IOL holder includes an integral adaptive feature that comprises a cantilever design, wherein the integral adaptive feature is capable of being positioned at angles inward from, outward from, and/or level with the lid along the length of the integral adaptive feature. In various embodiments, the IOL holder includes an integral adaptive feature that is integrally connected to the lid by one or more flexible connections.

In various embodiments, the angle of the integral adaptive feature with respect to the lid is set during manufacturing such that the integral adaptive feature will exert a desired force on a lens body of an intraocular lens when an intraocular lens is positioned on the integral annular support of the base and a lid is in a closed position. In various embodiments, the length of the integral adaptive feature with respect to the lid is set during manufacturing such that the integral adaptive feature will exert a desired force on a lens body of an intraocular lens when an intraocular lens is positioned on the integral annular support of the base and a lid is in a closed position. In various embodiments, the width of the integral adaptive feature with respect to the lid is set during manufacturing such that the integral adaptive feature will exert a desired force on a lens body of an intraocular lens when an intraocular lens is positioned on the integral annular support of the base and a lid is in a closed position. In various embodiments, the thickness of the of the integral connection of the integral adaptive feature with to the lid is set during manufacturing such that the integral adaptive feature will exert a desired force on a lens body of an intraocular lens when an intraocular lens is positioned on the integral annular support of the base and a lid is in a closed position.

As one skilled in the art will appreciate, the design of the connection of the integral adaptive feature to the IOL holder will influence the force that is exerted on the lens body. In the embodiment of the cantilever design, fulcrum width, fulcrum thickness and lever length will influence force. Material selection and thickness of the connection(s) will also influence force.

In various embodiments, the IOL holder includes an integral adaptive feature that comprises an integral annular protrusion that exerts a force on a lens body of an intraocular lens when an intraocular lens is positioned on the integral annular support of the base and a lid is in a closed position. In various embodiments, the IOL holder includes an integral adaptive feature that comprises an integral annular protrusion that comprises a concave profile and is positioned to apply a desired force on an intraocular lens when an intraocular lens is placed in the integrated annular support of the base and the lid is in a closed position.

In various embodiments, the IOL holder includes an integral adaptive feature that comprises an integral annular recession that exerts a force on a lens body of an intraocular lens when an intraocular lens is positioned on the integral annular support of the base and a lid is in a closed position. In various embodiments, the IOL holder includes an integral adaptive feature that comprises an integral annular recession that comprises a concave profile and is positioned to apply a desired force on an intraocular lens when an intraocular lens is placed in the integrated annular support of the base and the lid is in a closed position.

In various embodiments, the IOL holder includes an integral adaptive feature that comprises an integral annular feature that is level with the lid and that exerts a force on a lens body of an intraocular lens when an intraocular lens is positioned on the integral annular support of the base and a lid is in a closed position. In various embodiments, the IOL holder includes an integral adaptive feature that comprises an integral annular feature that is level with the lid and that comprises a concave profile and is positioned to apply a desired force on an intraocular lens when an intraocular lens is placed in the integrated annular support of the base and the lid is in a closed position.

In various embodiments, the IOL holder includes an integral adaptive feature that exerts a force on a lens body of an intraocular lens when an intraocular lens is positioned on the integral annular support of the base and a lid is in a closed position, wherein the force is enough to hold an intraocular lens in place on an integral annular support of a base without causing damage to an intraocular lens.

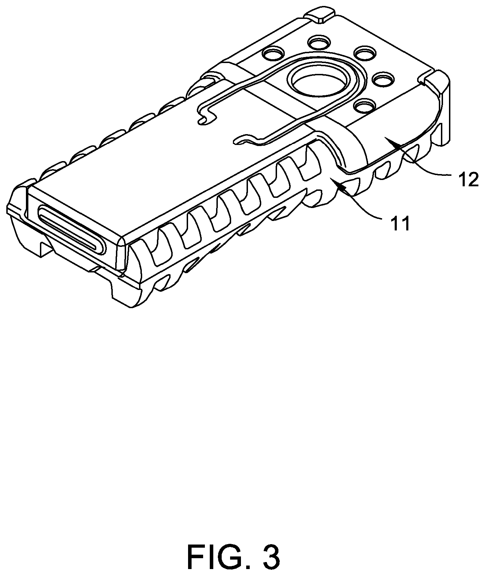

In various embodiments, as depicted in Figures 1 and 3, the IOL holder includes an integral adaptive feature **15** that comprises an integral annular protrusion **16** that applies a desired force on an intraocular lens when an intraocular lens is placed in the integrated annular support **14** of the base **11** and the lid **12** is in a closed position such that the force is enough to hold an intraocular lens **24** in place on an integral annular support of a base without causing damage to an intraocular lens **24**.

In various embodiments, the integral annular protrusion is designed to avoid significant contact with the optical surface of the lens. In further embodiments, the integral annular protrusion is designed to avoid contact with the optical surface of the lens. In still further embodiments, the integral annular protrusion is designed to avoid contact with the central portion of the optical surface of the lens within a 5 mm diameter. In still further embodiments, the integral annular protrusion is designed to avoid contact with the central portion of the optical surface of the lens within a 3 mm diameter.

In various embodiments, the IOL holder includes a base that comprises one or more integral posts positioned in proximity to an integral annular support of the base. In various embodiments the IOL holder includes one or more integral posts positioned at a distance from the integral annular support such that the haptics of an intraocular lens placed on the integrated annular support can make contact with the one or more integral posts.

In various embodiments, the IOL holder includes a base that comprises one or more integral posts positioned in proximity to an integral annular support of the base, wherein the one or more integral posts provide one or more contact points for one or more haptics when an intraocular lens is positioned on the integral annular support of the base. In various embodiments the haptics of an intraocular lens placed on the integrated annular support can make contact with the top of the one or more integral posts. In various embodiments the haptics of an intraocular lens placed on the integrated annular support can make contact with the sides of the one or more integral posts. In various embodiments the haptics of an intraocular lens placed on the integrated annular support can wrap at least partially around the sides of the one or more integral posts.

In various embodiments, the IOL holder includes a base that comprises one or more integral posts positioned in proximity to an integral annular support of the base, wherein the one or more integral posts comprise at least one recess feature. In various embodiments, the at least one recess feature is positioned on the top of the one or more integral posts. In various embodiments, the at least one recess feature is positioned on the side of the one or more integral posts.

In various embodiments, the IOL holder includes a base that comprises one or more integral posts positioned in proximity to an integral annular support of the base, wherein the one or more integral posts comprise at least one recess feature, wherein the at least one recess feature of the one or more integral posts provides one or more contact points for one or more haptics when an intraocular lens is positioned on the integral annular support of the base. In various embodiments, the haptics of an intraocular lens placed on the integrated annular support can make contact with the at least one recess feature that is positioned on the top of the one or more integral posts. In various embodiments, the haptics of an intraocular lens placed on the integrated annular support can make contact with the at least one recess feature that is positioned on the side of the one or more integral posts. In various embodiments, the haptics of an intraocular lens placed on the integrated annular support can make contact with the at least one recess feature that is positioned on the side of the one or more integral posts when the haptics wrap at least partially around the sides of the one or more integral posts.

In an embodiment of the invention depicted in Figure 1, the base **11** of the IOL holder comprises two integral posts **17** that each include at least one recess feature **18,** wherein the integral posts **17** are positioned at a distance from the integral annular support **14** of the base **11** such that the haptics **23** of a intraocular lens **24** placed on the integrated annular support **14** can make contact with the at least one recess feature **18** that is positioned on the top of each of the integral posts **17.**

An intraocular lens associated with an aspect of the invention may include a single piece intraocular lens or a three-piece intraocular lens, by way of non-limiting example.

In various embodiments, the IOL holder includes a lid that comprises one or more apertures to allow gas and/or liquid to flow through to an intraocular lens when an intraocular lens is positioned on the integral annular support of the base and the lid is in the closed position. In various embodiments, the one or more apertures included in the lid are arranged in a semicircular pattern positioned around the portion of the lid that is in close proximity to the end of the integral adaptive feature that exerts a force on a lens body of an intraocular lens when an intraocular lens is positioned on the integral annular support of the base and the lid is in a closed position. In various embodiments, the one or more apertures included in the lid are arranged in a linear pattern. In various embodiments, the one or more apertures included in the lid are arranged in a partial square or rectangular pattern. In various embodiments, the one or more apertures included in the lid are each circular, ovular, square, rectangular, or triangular in shape. In the embodiment of the invention shown in Fig. 1, the one or more apertures **19** included in the lid **12** are circular in shape and arranged in a semicircular pattern.

In various embodiments, the IOL holder includes a base that comprises one or more apertures to allow gas and/or liquid to flow through to an intraocular lens when an intraocular lens is positioned on the integral annular support of the base and the lid is in the closed position. In various embodiments, the one or more apertures included in the base are arranged in a linear pattern aligned from the end distal to the integral annular support and leading towards, but short of the integral annular support. In various embodiments, the one or more apertures included in the base are arranged in a diagonal pattern. In various embodiments, the one or more apertures included in the base are arranged in a circular, ovular, square, rectangular, or triangular pattern. In various embodiments, the one or more apertures included in the base are each circular, ovular, square, rectangular, or triangular in shape. In Fig. 1, the one or more apertures **20** included in the base **11** are rectangular in shape and arranged in a linear pattern.

In various embodiments, the IOL holder includes a lid that automatically interlocks with a base when the lid is in the closed position, wherein the interlocking of the lid and the base is reversible. In various embodiments, the IOL holder includes a lid that automatically interlocks with a base when the lid is in the closed position, wherein the interlocking of the lid and the base is reversible, wherein the lid comprises an interlocking recess, wherein the base comprises an interlocking protrusion, and wherein the interlocking recess and the interlocking protrusion provide a means for the interlocking of the lid and the base. In various embodiments, the IOL holder includes a lid that automatically interlocks with a base when the lid is in the closed position, wherein the interlocking of the lid and the base is reversible, wherein the lid comprises an interlocking protrusion, wherein the base comprises an interlocking recess, and wherein the interlocking protrusion and the interlocking recess provide a means for the interlocking of the lid and the base. In various embodiments, the IOL holder includes an integral latching mechanism that automatically interlocks and holds the lid and the base in a closed position, wherein the engagement of the latching mechanism can be reversed. In various embodiments, the IOL holder includes an interlocking latch on the lid and an integral post on the base that provide a means for the reversible interlocking of the lid and the base. In various embodiments, the IOL holder includes an interlocking latch on the base and an integral post on the lid that provide a means for the reversible interlocking of the lid and the base.

The reversibility of the interlocking of the lid and the base is meant to require the application of manual force approximating the manual force that can be generated by an adult human hand while the IOL holder is being held by such adult human. In the embodiment of the invention depicted in Figures 1 and 3, the lid **12** of the IOL holder comprises an interlocking recess **21,** the base **11** comprises an interlocking protrusion **22,** and when the IOL holder is in the closed position, the interlocking recess **21** and the interlocking protrusion **22** provide a means for the automatic and reversible interlocking of the lid and the base.

FIG. 1 illustrates an exemplary IOL holder disclosed herein displayed with a lid **12** in an open position and with a single piece intraocular lens **24** in place on the integral annular support **14** with the haptics **23** in contact with the recess features **18** of the integral posts **17.** This exemplary IOL holder comprises a one-piece device comprising a base **11,** a lid **12,** and two integral hinges **13** that connect the base **11** to the lid **12,** an integral annular support **14** protruding from the base **11,** an integral adaptive feature **15** of a cantilever design that is connected at one end to the lid **12,** an integral annular protrusion **16** protruding from the integral adaptive feature **15,** two integral posts **17** that each include a recess feature **18,** circular apertures **19** in the lid **12,** rectangular apertures **20** in the base **11,** and a means for interlocking the lid **12** and the base **11** including an interlocking recess **21** in the lid **12** and an interlocking protrusion **22** in the base **11.**

FIG. 2 illustrates the same exemplary IOL holder illustrated in FIG 1 displayed with the lid **12** in an open position with a multiple piece (3-piece) intraocular lens in place on the integral annular support **14** protruding from the base **11,** and the haptics **23** partially wrapped around the integral posts **17.**

FIG. 3 illustrates the same exemplary IOL holder illustrated in FIG 1 displayed with the lid **12** in a closed position upon the base **11.**

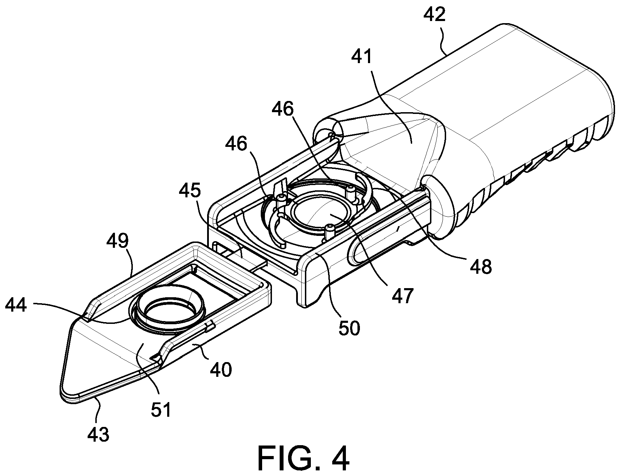

FIG. 4 illustrates an exemplary IOL holder disclosed herein displayed with a lid **40** in an open position and with a single piece intraocular lens **47** resting on the floor of the IOL holder body **42** with the haptics **48** in contact with the retention posts **46.** The retention posts **46** may be in contact the body of the intraocular lens **47** to suitably hold the intraocular lens **47** in a static position within the body **42** when lid **40** is in the open position. This exemplary IOL holder comprises a one-piece device comprising a body **42,** a lid **40,** and an integral hinge **45** that connect the body **42** to the lid **40,** an integral adaptive feature **51** of a cantilever design that is connected at one end to the lid **40,** an integral annular protrusion **44** protruding from the integral adaptive feature **51,** three retention posts **46,** and a means for the lid **40** and the body **42** including an interlocking sides **49** in the lid **40** and an interlocking protrusion **50** in the body **42.** The body **42** includes a recess **41** which allows tab **43** of the lid **40** to at least partially contact body **42.**

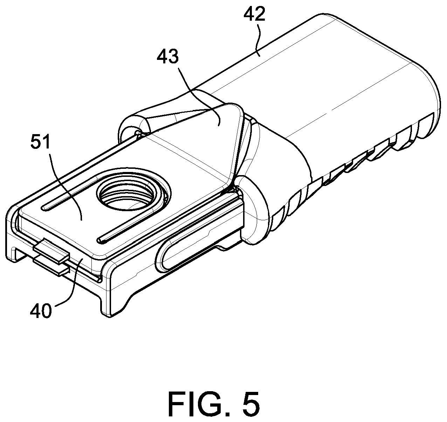

FIG. 5 illustrates the same exemplary IOL holder illustrated in FIG 4 displayed with the lid **40** in a closed position upon the body **42.**

Various embodiments of the disclosure further relate to a method of manufacture of an IOL holder comprising forming an IOL holder from at least one polymer, wherein the IOL holder comprises a one-piece device comprising a base, a lid, and at least one integral hinge that connects the base to the lid, wherein the base of the IOL holder comprises an integral annular support that is designed to accommodate an intraocular lens, and wherein the lid of the IOL holder comprises an integral adaptive feature that exerts a force on a lens body of an intraocular lens when an intraocular lens is positioned on the integral annular support of the base and the lid is in a closed position.

In various embodiments, the method of manufacture of an IOL holder comprises forming an IOL holder from at least one polymer, wherein the at least one polymer is molded. In various embodiments, the method of manufacture of an IOL holder comprises forming an IOL holder from at least one polymer, wherein the at least one polymer is printed. Suitable polymers include those that are compatible with the material of construction of an intraocular lens and the methods of use of the IOL holder including, but not limited to, thermal plastics, biopolymers, and copolymers. More specifically, the polymers include, but are not limited to, polyolefins (e.g., polypropylene and polyethylene), polycarbonate, acrylic polymers, acrylic copolymers, nylon, polyester, cellulose acetate, acetate/butyrate, and the like. Preferably, the method of manufacture comprises molding or printing the IOL holder from polypropylene.

In various embodiments, the method of manufacture of an IOL holder comprises forming an IOL holder that can accommodate various types, models, shapes, and sizes of a single piece or a multiple piece intraocular lens.

In various embodiments, the method of manufacture of an IOL holder comprises forming an IOL holder that includes an integral annular support that is designed to protrude from the base of the IOL holder as a column comprising a concave profile at the distal end of the integral annular support with respect to the base. In various embodiments, the method of manufacture of an IOL holder comprises forming an IOL holder that includes an integral annular support that is designed to be level with the base of the IOL holder and comprise a concave profile. In various embodiments, the method of manufacture of an IOL holder comprises forming an IOL holder that includes an integral annular support that is designed to recede within the base, of the IOL holder and comprise a concave profile. Preferably, the method of manufacture comprises forming an IOL holder that includes an integral annular support that protrudes from the base in a columnar shape and comprises a concave profile at the distal end of the integral annular support with respect to the base.

In various embodiments, the method of manufacture of an IOL holder comprises forming an IOL holder that includes an integral adaptive feature that comprises a cantilever design, wherein only one end of the integral adaptive feature is integrally connected to the lid. In various embodiments, the method of manufacture of an IOL holder comprises forming an IOL holder that includes an integral adaptive feature that comprises a cantilever design, wherein the integral adaptive feature is capable of being positioned at angles inward from, outward from, and/or level with the lid along the length of the integral adaptive feature.

In various embodiments, the method of manufacture of an IOL holder comprises forming an IOL holder that includes an angle of the integral adaptive feature with respect to the lid that is set during manufacturing such that the integral adaptive feature will exert a desired force on a lens body of an intraocular lens when an intraocular lens is positioned on the integral annular support of the base and a lid is in a closed position. In various embodiments, the method of manufacture of an IOL holder comprises forming an IOL holder that includes a length of the integral adaptive feature with respect to the lid that is set during manufacturing such that the integral adaptive feature will exert a desired force on a lens body of an intraocular lens when an intraocular lens is positioned on the integral annular support of the base and a lid is in a closed position. In various embodiments, the method of manufacture of an IOL holder comprises forming an IOL holder that includes a width of the integral adaptive feature with respect to the lid that is set during manufacturing such that the integral adaptive feature will exert a desired force on a lens body of an intraocular lens when an intraocular lens is positioned on the integral annular support of the base and a lid is in a closed position. In various embodiments, the method of manufacture of an IOL holder comprises forming an IOL holder that includes a thickness of the of the integral connection of the integral adaptive feature with to the lid that is set during manufacturing such that the integral adaptive feature will exert a desired force on a lens body of an intraocular lens when an intraocular lens is positioned on the integral annular support of the base and a lid is in a closed position.

In various embodiments, the method of manufacture of an IOL holder comprises forming an IOL holder that includes an integral adaptive feature that comprises an integral annular protrusion that exerts a force on a lens body of an intraocular lens when an intraocular lens is positioned on the integral annular support of the base and a lid is in a closed position. In various embodiments, the method of manufacture of an IOL holder comprises forming an IOL holder that includes an integral adaptive feature that comprises an integral annular protrusion that comprises a concave profile and is positioned to apply a desired force on an intraocular lens when an intraocular lens is placed in the integrated annular support of the base and the lid is in a closed position. A desired force in this situation is one that would generally maintain the intraocular lens in position, but would not deform or damage the intraocular lens when the intraocular lens is placed in the integrated annular support of the base and the lid is in a closed position.

In various embodiments, the method of manufacture of an IOL holder comprises forming an IOL holder that includes an integral adaptive feature that comprises an integral annular recession that exerts a force on a lens body of an intraocular lens when an intraocular lens is positioned on the integral annular support of the base and a lid is in a closed position. In various embodiments, the method of manufacture of an IOL holder comprises forming an IOL holder that includes an integral adaptive feature that comprises an integral annular recession that comprises a concave profile and is positioned to apply a desired force on an intraocular lens when an intraocular lens is placed in the integrated annular support of the base and the lid is in a closed position.

In various embodiments, the method of manufacture of an IOL holder comprises forming an IOL holder that includes an integral adaptive feature that comprises an integral annular feature that is level with the lid and that exerts a force on a lens body of an intraocular lens when an intraocular lens is positioned on the integral annular support of the base and a lid is in a closed position. In various embodiments, the method of manufacture of an IOL holder comprises forming an IOL holder that includes an integral adaptive feature that comprises an integral annular feature that is level with the lid and that comprises a concave profile and is positioned to apply a desired force on an intraocular lens when an intraocular lens is placed in the integrated annular support of the base and the lid is in a closed position.

In various embodiments, the method of manufacture of an IOL holder comprises forming an IOL holder that includes an integral adaptive feature that exerts a force on a lens body of an intraocular lens when an intraocular lens is positioned on the integral annular support of the base and a lid is in a closed position, wherein the force is enough to hold an intraocular lens in place on an integral annular support of a base without causing damage to an intraocular lens.

Preferably, the method of manufacture of an IOL holder comprises forming an IOL holder that includes an integral adaptive feature that comprises an integral annular protrusion that applies a desired force on an intraocular lens when an intraocular lens is placed in the integrated annular support of the base and the lid is in a closed position such that the force is enough to hold an intraocular lens in place on an integral annular support of a base without causing damage to an intraocular lens.

In various embodiments, the method of manufacture of an IOL holder comprises forming an IOL holder that includes a base that comprises one or more integral posts positioned in proximity to an integral annular support of the base. In various embodiments, the method of manufacture of an IOL holder comprises forming an IOL holder that includes one or more integral posts positioned at a distance from the integral annular support such that the haptics of an intraocular lens placed on the integrated annular support can make contact with the one or more integral posts.

In various embodiments, the method of manufacture of an IOL holder comprises forming an IOL holder that includes a base that comprises one or more integral posts positioned in proximity to an integral annular support of the base, wherein the one or more integral posts provide one or more contact points for one or more haptics when an intraocular lens is positioned on the integral annular support of the base. In various embodiments, the method of manufacture of an IOL holder comprises forming an IOL holder wherein the haptics of an intraocular lens placed on the integrated annular support can make contact with the top of the one or more integral posts. In various embodiments, the method of manufacture of an IOL holder comprises forming an IOL holder wherein the haptics of an intraocular lens placed on the integrated annular support can make contact with the sides of the one or more integral posts. In various embodiments, the method of manufacture of an IOL holder comprises forming an IOL holder wherein the haptics of an intraocular lens placed on the integrated annular support can wrap at least partially around the sides of the one or more integral posts.

In various embodiments, the method of manufacture of an IOL holder comprises forming an IOL holder that includes a base that comprises one or more integral posts positioned in proximity to an integral annular support of the base, wherein the one or more integral posts comprise at least one recess feature. In various embodiments, the method of manufacture of an IOL holder comprises forming an IOL holder wherein the at least one recess feature is positioned on the top of the one or more integral posts. In various embodiments, the method of manufacture of an IOL holder comprises forming an IOL holder wherein the at least one recess feature is positioned on the side of the one or more integral posts.

In various embodiments, the method of manufacture of an IOL holder comprises forming an IOL holder that includes a base that comprises one or more integral posts positioned in proximity to an integral annular support of the base, wherein the one or more integral posts comprise at least one recess feature, wherein the at least one recess feature of the one or more integral posts provides one or more contact points for one or more haptics when an intraocular lens is positioned on the integral annular support of the base. In various embodiments, the method of manufacture of an IOL holder comprises forming an IOL holder wherein the haptics of an intraocular lens placed on the integrated annular support can make contact with the at least one recess feature that is positioned on the top of the one or more integral posts. In various embodiments, the method of manufacture of an IOL holder comprises forming an IOL holder wherein the haptics of an intraocular lens placed on the integrated annular support can make contact with the at least one recess feature that is positioned on the side of the one or more integral posts. In various embodiments, the method of manufacture of an IOL holder comprises forming an IOL holder wherein the haptics of an intraocular lens placed on the integrated annular support can make contact with the at least one recess feature that is positioned on the side of the one or more integral posts when the haptics wrap at least partially around the sides of the one or more integral posts.

Preferentially, the method of manufacture of an IOL holder comprises forming an IOL holder wherein the base of the IOL holder comprises two integral posts that each include at least one recess feature, wherein the integral posts are positioned at a distance from the integral annular support of the base such that the haptics of a single piece intraocular lens placed on the integrated annular support can make contact with the at least one recess feature that is positioned on the top of each of the integral posts, and wherein the haptics of a three piece intraocular lens placed on the integrated annular support can at least partially wrap around and contact the sides of the integral posts.

In various embodiments, the method of manufacture of an IOL holder comprises forming an IOL holder that includes a lid that comprises one or more apertures to allow gas and/or liquid to flow through to an intraocular lens when an intraocular lens is positioned on the integral annular support of the base and the lid is in the closed position. In various embodiments, the method of manufacture of an IOL holder comprises forming an IOL holder wherein the one or more apertures included in the lid are arranged in a semicircular pattern positioned around the portion of the lid that is in close proximity to the end of the integral adaptive feature that exerts a force on a lens body of an intraocular lens when an intraocular lens is positioned on the integral annular support of the base and the lid is in a closed position. In various embodiments, the method of manufacture of an IOL holder comprises forming an IOL holder wherein the one or more apertures included in the lid are arranged in a linear pattern. In various embodiments, the method of manufacture of an IOL holder comprises forming an IOL holder wherein the one or more apertures included in the lid are arranged in a partial square or rectangular pattern. In various embodiments, the method of manufacture of an IOL holder comprises forming an IOL holder wherein the one or more apertures included in the lid are each circular, ovular, square, rectangular, or triangular in shape. Preferentially, the method of manufacture of an IOL holder comprises forming an IOL holder wherein the one or more apertures included in the lid are circular in shape and arranged in a semicircular pattern.

In various embodiments, the method of manufacture of an IOL holder comprises forming an IOL holder that includes a base that comprises one or more apertures to allow gas and/or liquid to flow through to an intraocular lens when an intraocular lens is positioned on the integral annular support of the base and the lid is in the closed position. In various embodiments, the method of manufacture of an IOL holder comprises forming an IOL holder wherein the one or more apertures included in the base are arranged in a linear pattern aligned from the end distal to the integral annular support and leading towards, but short of the integral annular support. In various embodiments, the method of manufacture of an IOL holder comprises forming an IOL holder wherein the one or more apertures included in the base are arranged in a diagonal pattern. In various embodiments, the method of manufacture of an IOL holder comprises forming an IOL holder wherein the one or more apertures included in the base are arranged in a circular, ovular, square, rectangular, or triangular pattern. In various embodiments, the method of manufacture of an IOL holder comprises forming an IOL holder wherein the one or more apertures included in the base are each circular, ovular, square, rectangular, or triangular in shape. Preferentially, the method of manufacture of an IOL holder comprises forming an IOL holder wherein the one or more apertures included in the base are rectangular in shape and arranged in a linear pattern.

In various embodiments, the method of manufacture of an IOL holder comprises forming an IOL holder that includes a lid that automatically interlocks with a base when the lid is in the closed position, wherein the interlocking of the lid and the base is reversible. In various embodiments, the method of manufacture of an IOL holder comprises forming an IOL holder that includes a lid that automatically interlocks with a base when the lid is in the closed position, wherein the interlocking of the lid and the base is reversible, wherein the lid comprises an interlocking recess, wherein the base comprises an interlocking protrusion, and wherein the interlocking recess and the interlocking protrusion provide a means for the interlocking of the lid and the base. In various embodiments, the method of manufacture of an IOL holder comprises forming an IOL holder that includes a lid that automatically interlocks with a base when the lid is in the closed position, wherein the interlocking of the lid and the base is reversible, wherein the lid comprises an interlocking protrusion, wherein the base comprises an interlocking recess, and wherein the interlocking protrusion and the interlocking recess provide a means for the interlocking of the lid and the base. In various embodiments, the method of manufacture of an IOL holder comprises forming an IOL holder that includes an integral latching mechanism that automatically interlocks and holds the lid and the base in a closed position, wherein the engagement of the latching mechanism can be reversed. In various embodiments, the method of manufacture of an IOL holder comprises forming an IOL holder that includes an interlocking latch on the lid and an integral post on the base that provide a means for the reversible interlocking of the lid and the base. In various embodiments, the method of manufacture of an IOL holder comprises forming an IOL holder that includes an interlocking latch on the base and an integral post on the lid that provide a means for the reversible interlocking of the lid and the base.

The reversibility of the interlocking of the lid and the base is meant to require the application of manual force approximating the manual force that can be generated by an adult human hand while the IOL holder is being held by such adult human. Preferentially, the method of manufacture of an IOL holder comprises forming an IOL holder wherein the lid of the IOL holder comprises an interlocking recess, the base comprises an interlocking protrusion, and when the IOL holder is in the closed position, the interlocking recess and the interlocking protrusion provide a means for the automatic and reversible interlocking of the lid and the base.

Various embodiments of the disclosure further relate to a method of use of an IOL holder comprising holding, transporting, shipping, carrying, washing, sterilizing, aerating, degassing, and/or storing an intraocular lens in the IOL holder, wherein the IOL holder comprises a one-piece device comprising a base, a lid, and at least one integral hinge that connects the base to the lid, wherein the base of the IOL holder comprises an integral annular support that is designed to accommodate an intraocular lens, and wherein the lid of the IOL holder comprises an integral adaptive feature that exerts a force on a lens body of an intraocular lens when an intraocular lens is positioned on the integral annular support of the base and the lid is in a closed position.

In various embodiments, the method of use comprises holding and/or storing an intraocular lens in the IOL holder, wherein the intraocular lens is held and/or stored in a substantially unfolded state such that the haptics are substantially in their natural position with respect to the optic. In various embodiments, the method of use comprises holding and/or storing an intraocular lens in the IOL holder, wherein the intraocular lens is held and/or stored in a partially folded state such that the haptics are partially folded towards, above, and/or below the optic. In various embodiments, the method of use comprises holding and/or storing an intraocular lens in the IOL holder, wherein the intraocular lens is held and/or stored in a substantially folded state such that the haptics are substantially folded towards, above, and/or below the optic. Preferentially, the IOL holder holds an intraocular lens in a substantially unfolded state.

In various embodiments, the method of use comprises washing, sterilizing, aerating, and/or degassing an intraocular lens in the IOL holder, wherein the intraocular lens is substantially washed, sterilized, aerated, and/or degassed using techniques and procedures that are known to be compatible with an intraocular lens. In various embodiments, the method of use comprises transporting, shipping, and/or carrying an intraocular lens in the IOL holder, wherein the intraocular lens is transported, shipped, and/or carried using techniques and procedures that are known to be compatible with an intraocular lens.

In various embodiments, the method of use of an IOL holder comprises using an IOL holder that is formed from at least one polymer, wherein the at least one polymer is molded. In various embodiments, the method of use of an IOL holder comprises using an IOL holder that is formed from at least one polymer, wherein the at least one polymer is printed. Suitable polymers include those that are compatible with the material of construction of an intraocular lens and the methods of use of the IOL holder including, but not limited to, thermal plastics, biopolymers, and copolymers. More specifically, the polymers include, but are not limited to, polyolefins (e.g., polypropylene and polyethylene), polycarbonate, acrylic polymers, acrylic copolymers, nylon, polyester, cellulose acetate, acetate/butyrate, and the like. Preferably, the method of manufacture comprises molding or printing the IOL holder from polypropylene.

In various embodiments, the method of use of an IOL holder comprises using an IOL holder that can accommodate various types, models, shapes, and sizes of a single piece or a multiple piece intraocular lens.

In various embodiments, the method of use of an IOL holder comprises using an IOL holder that includes an integral annular support that is designed to protrude from the base of the IOL holder as a column comprising a concave profile at the distal end of the integral annular support with respect to the base. In various embodiments, the method of use of an IOL holder comprises using an IOL holder that includes an integral annular support that is designed to be level with the base of the IOL holder and comprise a concave profile. In various embodiments, the method of use of an IOL holder comprises using an IOL holder that includes an integral annular support that is designed to recede within the base, of the IOL holder and comprise a concave profile. Preferably, the method of use of an IOL holder comprises using an IOL holder that includes an integral annular support that protrudes from the base in a columnar shape and comprises a concave profile at the distal end of the integral annular support with respect to the base.

In various embodiments, the method of use of an IOL holder comprises using an IOL holder that includes an integral adaptive feature that comprises a cantilever design, wherein only one end of the integral adaptive feature is integrally connected to the lid. In various embodiments, the method of use of an IOL holder comprises using an IOL holder that includes an integral adaptive feature that comprises a cantilever design, wherein the integral adaptive feature is capable of being positioned at angles inward from, outward from, and/or level with the lid along the length of the integral adaptive feature.

In various embodiments, the method of use of an IOL holder comprises using an IOL holder that includes an angle of the integral adaptive feature with respect to the lid that is set during manufacturing such that the integral adaptive feature will exert a desired force on a lens body of an intraocular lens when an intraocular lens is positioned on the integral annular support of the base and a lid is in a closed position. In various embodiments, the method of use of an IOL holder comprises using an IOL holder that includes a length of the integral adaptive feature with respect to the lid that is set during manufacturing such that the integral adaptive feature will exert a desired force on a lens body of an intraocular lens when an intraocular lens is positioned on the integral annular support of the base and a lid is in a closed position. In various embodiments, the method of use of an IOL holder comprises using an IOL holder that includes a width of the integral adaptive feature with respect to the lid that is set during manufacturing such that the integral adaptive feature will exert a desired force on a lens body of an intraocular lens when an intraocular lens is positioned on the integral annular support of the base and a lid is in a closed position. In various embodiments, the method of use of an IOL holder comprises using an IOL holder that includes a thickness of the of the integral connection of the integral adaptive feature with to the lid that is set during manufacturing such that the integral adaptive feature will exert a desired force on a lens body of an intraocular lens when an intraocular lens is positioned on the integral annular support of the base and a lid is in a closed position.

In various embodiments, the method of use of an IOL holder comprises using an IOL holder that includes an integral adaptive feature that comprises an integral annular protrusion that exerts a force on a lens body of an intraocular lens when an intraocular lens is positioned on the integral annular support of the base and a lid is in a closed position. In various embodiments, the method of use of an IOL holder comprises using an IOL holder that includes an integral adaptive feature that comprises an integral annular protrusion that comprises a concave profile and is positioned to apply a desired force on an intraocular lens when an intraocular lens is placed in the integrated annular support of the base and the lid is in a closed position.

In various embodiments, the method of use of an IOL holder comprises using an IOL holder that includes an integral adaptive feature that comprises an integral annular recession that exerts a force on a lens body of an intraocular lens when an intraocular lens is positioned on the integral annular support of the base and a lid is in a closed position. In various embodiments, the method of use of an IOL holder comprises using an IOL holder that includes an integral adaptive feature that comprises an integral annular recession that comprises a concave profile and is positioned to apply a desired force on an intraocular lens when an intraocular lens is placed in the integrated annular support of the base and the lid is in a closed position.

In various embodiments, the method of use of an IOL holder comprises using an IOL holder that includes an integral adaptive feature that comprises an integral annular feature that is level with the lid and that exerts a force on a lens body of an intraocular lens when an intraocular lens is positioned on the integral annular support of the base and a lid is in a closed position. In various embodiments, the method of use of an IOL holder comprises using an IOL holder that includes an integral adaptive feature that comprises an integral annular feature that is level with the lid and that comprises a concave profile and is positioned to apply a desired force on an intraocular lens when an intraocular lens is placed in the integrated annular support of the base and the lid is in a closed position.

In various embodiments, the method of use of an IOL holder comprises using an IOL holder that includes an integral adaptive feature that exerts a force on a lens body of an intraocular lens when an intraocular lens is positioned on the integral annular support of the base and a lid is in a closed position, wherein the force is enough to hold an intraocular lens in place on an integral annular support of a base without causing damage to an intraocular lens.

Preferably, the method of use of an IOL holder comprises using an IOL holder that includes an integral adaptive feature that comprises an integral annular protrusion that applies a desired force on an intraocular lens when an intraocular lens is placed in the integrated annular support of the base and the lid is in a closed position such that the force is enough to hold an intraocular lens in place on an integral annular support of a base without causing damage to an intraocular lens.

In various embodiments, the method of use of an IOL holder comprises using an IOL holder that includes a base that comprises one or more integral posts positioned in proximity to an integral annular support of the base. In various embodiments, the method of use of an IOL holder comprises using an IOL holder that includes one or more integral posts positioned at a distance from the integral annular support such that the haptics of an intraocular lens placed on the integrated annular support can make contact with the one or more integral posts.

In various embodiments, the method of use of an IOL holder comprises using an IOL holder that includes a base that comprises one or more integral posts positioned in proximity to an integral annular support of the base, wherein the one or more integral posts provide one or more contact points for one or more haptics when an intraocular lens is positioned on the integral annular support of the base. In various embodiments, the method of use of an IOL holder comprises using an IOL holder wherein the haptics of an intraocular lens placed on the integrated annular support can make contact with the top of the one or more integral posts. In various embodiments, the method of use of an IOL holder comprises using an IOL holder wherein the haptics of an intraocular lens placed on the integrated annular support can make contact with the sides of the one or more integral posts. In various embodiments, the method of use of an IOL holder comprises using an IOL holder wherein the haptics of an intraocular lens placed on the integrated annular support can wrap at least partially around the sides of the one or more integral posts.

In various embodiments, the method of use of an IOL holder comprises using an IOL holder that includes a base that comprises one or more integral posts positioned in proximity to an integral annular support of the base, wherein the one or more integral posts comprise at least one recess feature. In various embodiments, the method of use of an IOL holder comprises using an IOL holder wherein the at least one recess feature is positioned on the top of the one or more integral posts. In various embodiments, the method of use of an IOL holder comprises using an IOL holder wherein the at least one recess feature is positioned on the side of the one or more integral posts.

In various embodiments, the method of use of an IOL holder comprises using an IOL holder that includes a base that comprises one or more integral posts positioned in proximity to an integral annular support of the base, wherein the one or more integral posts comprise at least one recess feature, wherein the at least one recess feature of the one or more integral posts provides one or more contact points for one or more haptics when an intraocular lens is positioned on the integral annular support of the base. In various embodiments, the method of use of an IOL holder comprises using an IOL holder wherein the haptics of an intraocular lens placed on the integrated annular support can make contact with the at least one recess feature that is positioned on the top of the one or more integral posts. In various embodiments, the method of use of an IOL holder comprises using an IOL holder wherein the haptics of an intraocular lens placed on the integrated annular support can make contact with the at least one recess feature that is positioned on the side of the one or more integral posts. In various embodiments, the method of use of an IOL holder comprises using an IOL holder wherein the haptics of an intraocular lens placed on the integrated annular support can make contact with the at least one recess feature that is positioned on the side of the one or more integral posts when the haptics wrap at least partially around the sides of the one or more integral posts.

Preferentially, the method of use of an IOL holder comprises using an IOL holder wherein the base of the IOL holder comprises two integral posts that each include at least one recess feature, wherein the integral posts are positioned at a distance from the integral annular support of the base such that the haptics of a single piece intraocular lens placed on the integrated annular support can make contact with the at least one recess feature that is positioned on the top of each of the integral posts, and wherein the haptics of a three piece intraocular lens placed on the integrated annular support can at least partially wrap around and contact the sides of the integral posts.

In various embodiments, the method of use of an IOL holder comprises using an IOL holder that includes a lid that comprises one or more apertures to allow gas and/or liquid to flow through to an intraocular lens when an intraocular lens is positioned on the integral annular support of the base and the lid is in the closed position. In various embodiments, the method of use of an IOL holder comprises using an IOL holder wherein the one or more apertures included in the lid are arranged in a semicircular pattern positioned around the portion of the lid that is in close proximity to the end of the integral adaptive feature that exerts a force on a lens body of an intraocular lens when an intraocular lens is positioned on the integral annular support of the base and the lid is in a closed position. In various embodiments, the method of use of an IOL holder comprises using an IOL holder wherein the one or more apertures included in the lid are arranged in a linear pattern. In various embodiments, the method of use of an IOL holder comprises using an IOL holder wherein the one or more apertures included in the lid are arranged in a partial square or rectangular pattern. In various embodiments, the method of use of an IOL holder comprises using an IOL holder wherein the one or more apertures included in the lid are each circular, ovular, square, rectangular, or triangular in shape. Preferentially, the method of use of an IOL holder comprises using an IOL holder wherein the one or more apertures included in the lid are circular in shape and arranged in a semicircular pattern.

In various embodiments, the method of use of an IOL holder comprises using an IOL holder that includes a base that comprises one or more apertures to allow gas and/or liquid to flow through to an intraocular lens when an intraocular lens is positioned on the integral annular support of the base and the lid is in the closed position. In various embodiments, the method of use of an IOL holder comprises using an IOL holder wherein the one or more apertures included in the base are arranged in a linear pattern aligned from the end distal to the integral annular support and leading towards, but short of the integral annular support. In various embodiments, the method of use of an IOL holder comprises using an IOL holder wherein the one or more apertures included in the base are arranged in a diagonal pattern. In various embodiments, the method of use of an IOL holder comprises using an IOL holder wherein the one or more apertures included in the base are arranged in a circular, ovular, square, rectangular, or triangular pattern. In various embodiments, the method of use of an IOL holder comprises using an IOL holder wherein the one or more apertures included in the base are each circular, ovular, square, rectangular, or triangular in shape. Preferentially, the method of use of an IOL holder comprises using an IOL holder wherein the one or more apertures included in the base are rectangular in shape and arranged in a linear pattern.

In various embodiments, the method of use of an IOL holder comprises using an IOL holder that includes a lid that automatically interlocks with a base when the lid is in the closed position, wherein the interlocking of the lid and the base is reversible. IIn various embodiments, the method of use of an IOL holder comprises using an IOL holder that includes a lid that automatically interlocks with a base when the lid is in the closed position, wherein the interlocking of the lid and the base is reversible, wherein the lid comprises an interlocking recess, wherein the base comprises an interlocking protrusion, and wherein the interlocking recess and the interlocking protrusion provide a means for the interlocking of the lid and the base. In various embodiments, the method of use of an IOL holder comprises using an IOL holder that includes a lid that automatically interlocks with a base when the lid is in the closed position, wherein the interlocking of the lid and the base is reversible, wherein the lid comprises an interlocking protrusion, wherein the base comprises an interlocking recess, and wherein the interlocking protrusion and the interlocking recess provide a means for the interlocking of the lid and the base. In various embodiments, the method of use of an IOL holder comprises using an IOL holder that includes an integral latching mechanism that automatically interlocks and holds the lid and the base in a closed position, wherein the engagement of the latching mechanism can be reversed. In various embodiments, the method of use of an IOL holder comprises using an IOL holder that includes an interlocking latch on the lid and an integral post on the base that provide a means for the reversible interlocking of the lid and the base. In various embodiments, the method of use of an IOL holder comprises using an IOL holder that includes an interlocking latch on the base and an integral post on the lid that provide a means for the reversible interlocking of the lid and the base.

The reversibility of the interlocking of the lid and the base is meant to require the application of manual force approximating the manual force that can be generated by an adult human hand while the IOL holder is being held by such adult human. Preferentially, the method of use of an IOL holder comprises using an IOL holder wherein the lid of the IOL holder comprises an interlocking recess, the base comprises an interlocking protrusion, and when the IOL holder is in the closed position, the interlocking recess and the interlocking protrusion provide a means for the automatic and reversible interlocking of the lid and the base.

## Claims

1. A one-piece intraocular lens (IOL) holder comprising a first portion (11, 42), a second portion (12, 40), and at least one integral hinge (13, 45) connecting the first and second portions, the holder having a first, open position, and a second, closed position,
wherein the first portion comprises an integral annular support (14, 46) configured to accommodate a lens body of an intraocular lens (24, 47), and
wherein the second portion comprises an integral adaptive feature (15, 51) configured to exert a force on the intraocular lens positioned on the integral annular support of the first portion when the holder in in the second position.

2. The IOL holder according to claim 1, wherein the integral adaptive feature is configured to exert a force on the lens body of the intraocular lens when the holder in in the second position.

3. The IOL holder according to claim 1, wherein the integral adaptive feature is configured to exert a force on a haptic portion of the intraocular lens when the holder in in the second position.

4. The IOL holder according to claim 1, further comprising an intraocular lens positioned on the integral annular support.

5. The IOL holder of claim 1, wherein the integral adaptive feature comprises a cantilever design, wherein only one portion of the integral adaptive feature is integrally connected to the second portion.

6. The IOL holder of claim 1, wherein the integral adaptive feature is connected to the second portion by a flexible connection.

7. The IOL holder of claim 1, wherein the integral adaptive feature comprises an integral annular protrusion (16, 44) that exerts the force on a lens body of an intraocular lens when an intraocular lens is positioned on the integral annular support and opposing one of the top portion or lower portion is in a closed position.

8. The IOL holder of claim 1, wherein the force is sufficient to hold an intraocular lens in place on the integral annular support of the base when the holder is in the second position without causing damage to the intraocular lens.

9. The IOL holder of claim 1, wherein the first portion comprises one or more integral posts (17, 46) positioned in proximity to the integral annular support and wherein the one or more integral posts provide one or more contact points for one or more haptics (23, 48) when the intraocular lens is positioned on the integral annular support.

10. The IOL holder of claim 9, wherein the one or more integral posts comprise at least one recess feature.

11. The IOL holder of claim 10, wherein the at least one recess feature of the one or more integral posts provides one or more contact points for one or more haptics when the intraocular lens is positioned on the integral annular support.

12. The IOL holder of claim 1, wherein one of the first portion or second portion comprises one or more apertures (19, 20) to allow gas and/or liquid to pass through when the holder is in the second position.

13. The IOL holder of claim 1, wherein the first portion interlocks with the second portion when in the second position.

14. The IOL holder of claim 13, wherein the interlocking of the first portion and the second portion is reversible.

15. The IOL holder of claim 1, wherein one of the first portion and the second portion comprises an interlocking recess (21,41) and wherein the other of the first portion and the second portion comprises an interlocking protrusion (22,43), and wherein the interlocking recess and the interlocking protrusion provide a means for the interlocking of the first portion and the second portion.

16. The IOL holder of claim 1, wherein one of the first portion and the second portion comprises a first segment of an integrally-formed latch capable of interlocking with a second segment of the integrally-formed latch which is integrally-formed with the other of the first portion and the second portion.

17. A method of manufacture of an IOL holder comprising: forming the IOL holder of claim 1 from at least one polymer, by molding or printing the IOL holder from the polymer, preferably wherein the polymer is polypropylene.

## Patentansprüche

1. Einteiliger Halter für Intraokularlinsen (IOL-Halter), umfassend einen ersten Abschnitt (11, 42), einen zweiten Abschnitt (12, 40) und mindestens ein integrales Scharnier (13, 45), das den ersten und den zweiten Abschnitt verbindet, wobei der Halter eine erste, offene Position und eine zweite, geschlossene Position aufweist,
wobei der erste Abschnitt einen integralen ringförmigen Träger (14, 46) umfasst, der konfiguriert ist, um einen Linsenkörper einer Intraokularlinse (24, 47) aufzunehmen, und
wobei der zweite Abschnitt ein integrales adaptives Merkmal (15, 51) umfasst, das konfiguriert ist, um eine Kraft auf die Intraokularlinse auszuüben, die auf dem integralen ringförmigen Träger des ersten Abschnitts positioniert ist, wenn sich der Halter in der zweiten Position befindet.

2. IOL-Halter nach Anspruch 1, wobei das integrale adaptive Merkmal konfiguriert ist, um eine Kraft auf den Linsenkörper der Intraokularlinse auszuüben, wenn sich der Halter in der zweiten Position befindet.

3. IOL-Halter nach Anspruch 1, wobei das integrale adaptive Merkmal konfiguriert ist, um eine Kraft auf einen haptischen Abschnitt der Intraokularlinse auszuüben, wenn sich der Halter in der zweiten Position befindet.

4. IOL-Halter nach Anspruch 1, ferner umfassend eine Intraokularlinse, die auf dem integralen ringförmigen Träger positioniert ist.

5. IOL-Halter nach Anspruch 1, wobei das integrale adaptive Merkmal eine freitragende Konstruktion umfasst, wobei nur ein Abschnitt des integralen adaptiven Merkmals mit dem zweiten Abschnitt integral verbunden ist.

6. IOL-Halter nach Anspruch 1, wobei das integrale adaptive Merkmal durch eine flexible Verbindung mit dem zweiten Abschnitt verbunden ist.

7. IOL-Halter nach Anspruch 1, wobei das integrale adaptive Merkmal einen integralen ringförmigen Vorsprung (16, 44) umfasst, der die Kraft auf einen Linsenkörper einer Intraokularlinse ausübt, wenn eine Intraokularlinse auf dem integralen ringförmigen Träger positioniert ist und der gegenüberliegende eine von dem oberen Abschnitt oder dem unteren Abschnitt sich in einer geschlossenen Position befindet.

8. IOL-Halter nach Anspruch 1, wobei die Kraft ausreicht, um eine Intraokularlinse an ihrem Platz auf dem integralen ringförmigen Träger der Basis zu halten, wenn sich der Halter in der zweiten Position befindet, ohne die Intraokularlinse zu beschädigen.

9. IOL-Halter nach Anspruch 1, wobei der erste Abschnitt eine oder mehrere integrale Stützen (17, 46) umfasst, die in der Nähe des integralen ringförmigen Trägers positioniert sind, und wobei der eine oder die mehreren integralen Stützen einen oder mehrere Kontaktpunkte für eine oder mehrere Haptiken (23, 48) bereitstellen, wenn die Intraokularlinse auf dem integralen ringförmigen Träger positioniert ist.

10. IOL-Halter nach Anspruch 9, wobei die eine oder die mehreren integralen Stützen mindestens ein Aussparungsmerkmal umfassen.

11. IOL-Halter nach Anspruch 10, wobei das mindestens eine Aussparungsmerkmal der einen oder der mehreren integralen Stützen einen oder mehrere Kontaktpunkte für eine oder mehrere Haptiken bereitstellt, wenn die Intraokularlinse auf dem integralen ringförmigen Träger positioniert ist.

12. IOL-Halter nach Anspruch 1, wobei einer von dem ersten Abschnitt oder dem zweiten Abschnitt eine oder mehrere Öffnungen (19, 20) umfasst, um Gas und/oder Flüssigkeit zu ermöglichen, hindurchzutreten, wenn sich der Halter in der zweiten Position befindet.

13. IOL-Halter nach Anspruch 1, wobei der erste Abschnitt mit dem zweiten Abschnitt verriegelt ist, wenn er sich in der zweiten Position befindet.

14. IOL-Halter nach Anspruch 13, wobei das Verriegeln des ersten Abschnitts und des zweiten Abschnitts reversibel ist.

15. IOL-Halter nach Anspruch 1, wobei einer von dem ersten Abschnitt und dem zweiten Abschnitt eine Verriegelungsaussparung (21,41) umfasst und wobei der andere von dem ersten Abschnitt und dem zweiten Abschnitt einen Verriegelungsvorsprung (22,43) umfasst und wobei die Verriegelungsaussparung und der Verriegelungsvorsprung ein Mittel zum Verriegeln des ersten Abschnitts und des zweiten Abschnitts bereitstellen.

16. IOL-Halter nach Anspruch 1, wobei einer von dem ersten Abschnitt und dem zweiten Abschnitt ein erstes Segment einer integral ausgebildeten Festhaltevorrichtung umfasst, die in der Lage ist, mit einem zweiten Segment der integral ausgebildeten Festhaltvorrichtung zu verriegeln, die mit dem anderen von dem ersten Abschnitt und dem zweiten Abschnitt integral ausgebildet ist.

17. Verfahren für eine Herstellung eines IOL-Halters, umfassend: Ausbilden des IOL-Halters nach Anspruch 1 aus mindestens einem Polymer durch Formen oder Drucken des IOL-Halters aus dem Polymer, vorzugsweise wobei das Polymer Polypropylen ist.

## Revendications

1. Support de lentille intraoculaire (IOL) monobloc comprenant une première partie (11, 42), une seconde partie (12, 40) et au moins une charnière solidaire (13, 45) reliant les première et seconde parties, le support ayant une première position ouverte, et une seconde position fermée,
dans lequel la première partie comprend un appui annulaire solidaire (14, 46) conçu pour recevoir un corps de lentille d'une lentille intraoculaire (24, 47), et
dans lequel la seconde partie comprend une caractéristique adaptative solidaire (15, 51) conçue pour exercer une force sur la lentille intraoculaire positionnée sur l'appui annulaire solidaire de la première partie lorsque le support est dans la seconde position.

2. Support d'IOL selon la revendication 1, dans lequel la caractéristique adaptative solidaire est conçue pour exercer une force sur le corps de lentille de la lentille intraoculaire lorsque le support est dans la seconde position.

3. Support d'IOL selon la revendication 1, dans lequel la caractéristique adaptative solidaire est conçue pour exercer une force sur une partie haptique de la lentille intraoculaire lorsque le support est dans la seconde position.

4. Support d'IOL selon la revendication 1, comprenant en outre une lentille intraoculaire positionnée sur l'appui annulaire solidaire.

5. Support d'IOL selon la revendication 1, dans lequel la caractéristique adaptative solidaire comprend une conception en porte-à-faux, dans lequel une seule partie de la caractéristique adaptative solidaire est reliée de façon solidaire à la seconde partie.

6. Support d'IOL selon la revendication 1, dans lequel la caractéristique adaptative solidaire est reliée à la seconde partie par une liaison flexible.

7. Support d'IOL selon la revendication 1, dans lequel la caractéristique adaptative solidaire comprend une saillie annulaire solidaire (16, 44) qui exerce la force sur un corps de lentille d'une lentille intraoculaire lorsqu'une lentille intraoculaire est positionnée sur l'appui annulaire solidaire et qu'une partie opposée à la partie supérieure ou la partie inférieure est dans une position fermée.

8. Support d'IOL selon la revendication 1, dans lequel la force est suffisante pour maintenir une lentille intraoculaire en place sur l'appui annulaire solidaire de la base lorsque le support est dans la seconde position sans provoquer de dommage à la lentille intraoculaire.

9. Support d'IOL selon la revendication 1, dans lequel la première partie comprend un ou plusieurs plots solidaires (17, 46) positionnés à proximité de l'appui annulaire solidaire et dans lequel le ou les plots solidaires fournissent un ou plusieurs points de contact pour une ou plusieurs haptiques (23, 48) lorsque la lentille intraoculaire est positionnée sur l'appui annulaire solidaire.

10. Support d'IOL selon la revendication 9, dans lequel le ou les plots solidaires comprennent au moins une caractéristique en retrait.

11. Support d'IOL selon la revendication 10, dans lequel l'au moins une caractéristique en retrait du ou des plots solidaires fournit un ou plusieurs points de contact pour une ou plusieurs haptiques lorsque la lentille intraoculaire est positionnée sur l'appui annulaire solidaire.

12. Support d'IOL selon la revendication 1, dans lequel l'une parmi la première partie ou la seconde partie comprend une ou plusieurs ouvertures (19, 20) pour permettre à un gaz et/ou à un liquide de traverser lorsque le support est dans la seconde position.

13. Support d'IOL selon la revendication 1, dans lequel la première partie s'enclenche réciproquement avec la seconde partie lorsqu'on se trouve dans la seconde position.

14. Support d'IOL selon la revendication 13, dans lequel l'enclenchement réciproque de la première partie et de la seconde partie est réversible.

15. Support d'IOL selon la revendication 1, dans lequel l'une parmi la première partie et la seconde partie comprend un retrait d'enclenchement réciproque (21, 41) et dans lequel l'autre parmi la première partie et la seconde partie comprend une saillie d'enclenchement réciproque (22, 43), et dans lequel le retrait d'enclenchement réciproque et la saillie d'enclenchement réciproque fournissent un moyen permettant l'enclenchement réciproque de la première partie et de la seconde partie.

16. Support d'IOL selon la revendication 1, dans lequel l'une parmi la première partie et la seconde partie comprend un premier segment d'un verrouillage formé de façon solidaire capable de s'enclencher réciproquement avec un second segment du verrouillage formé de façon solidaire qui est formé de façon solidaire avec l'autre parmi la première partie et la seconde partie.

17. Procédé de fabrication d'un support d'IOL comprenant : la formation du support d'IOL selon la revendication 1 à partir d'au moins un polymère, par moulage ou impression du support d'IOL à partir du polymère, de préférence dans lequel le polymère est du polypropylène.
